# EUROPEAN PATENT APPLICATION

(11) **EP 0 621 256 A1**
(43) Date of publication of application: **26.10.1994**
(21) Application number: 94201142.0
(22) Date of filing: 30.08.1991
(51) Int. Cl.: C07C 68/02, C07C 69/96, B01D 69/12, B01D 71/56, B01D 71/54

(54) **Chloroformyloxy-substituted isophthaloyl chlorides**

(30) Priority: 31.08.1990 US 576038
(62) Divisional of application: 91307999.2
(71) Applicant: E.I. DU PONT DE NEMOURS AND COMPANY, Wilmington Delaware 19898 (US)
(72) Inventor: Arthur, Samuel David, Wilmington, Delaware 19802 (US)
(74) Representative: Jones, Alan John

(57) **Abstract**

Chloroformyloxy-substituted isophthaloyl chlorides are made by forming a mixture of hydroxy-substituted isophthalic acid, or salt thereof, catalyst, phosgene and solvent, heating the mixture in a pressure vessel under autogeneous pressure, treating the mixture to remove the solvent, and distilling the mixture to yield the chloroformyloxy-substituted isophthaloyl chloride. The preferred chloroformyloxy-substituted isophthaloyl chloride is 5-chloroformyloxyisophthaloyl chloride and the preferred catalyst is imidazole.

## Description

This invention relates to the preparation of chloroformyloxy isophthaloyl chlorides.

According to this invention haloformyloxy-substituted isophthaloyl chlorides are prepared by reacting an hydroxy-substituted isophthalic acid, or a salt of hydroxy-substituted isophthalic acid, phosgene, and a solvent in the presence of a catalyst, under autogenous pressure at an elevated temperature, e.g. in a pressure vessel. The preferred hydroxy-substituted isophthalic acid is 5-hydroxyisophthalic acid and the preferred solvent is chlorinated benzene. Examples of chloroformyloxy-substituted isophthaloyl chlorides are 4-chloroformyloxyisophthaloyl chloride, 2-chloroformyloxyisophthaloyl chloride, and 5-chloroformyloxyisophthaloyl chloride.

5-chloroformyloxyisophthaloyl chloride (CFIC) also may be produced by using alternatives to the preferred reactants mentioned above. For example, salts of 5-hydroxyisophthalic acid such as disodium 5-hydroxyisophthalate or trisodium 5-hydroxyisophthalate may be substituted for 5-hydroxyisophthalic acid. Similarly, the catalyst imidazole, may be replaced with other heteroatom-containing compounds capable of complexing phosgene. Examples of such catalysts include, but are not limited to, pyridine, N,N-dimethylformamide (DMF), N,N-dimethylacetamide (DMAc), and the like. Likewise, solvents such as dioxane and methylene chloride may be employed, so long as the solvent is reasonably unreactive with the reactants and products.

The chloroformyl substituted isophthaloyl chlorides made by the process of the invention may be used in solution in a solvent to treat polyfunctional amine impregnated substrates which treated substrates may be used as reverse osmosis membranes. The specification of copending EP 91307999.2 describes a method of making reverse osmosis membranes wherein a polyfunctional amine treated substrate is treated with a haloformyloxy-substituted acyl halide.

### EXAMPLE

5-chloroformyloxyisophthaloyl chloride (CFIC) was prepared by heating a mixture of 25g of 5-hydroxyisophthalic acid, 0.3g of imidazole, 70g of phosgene, and 100ml of chlorobenzene solvent in a pressure vessel at 160°C for 18 hours under autogeneous pressure. Removal of the solvent, followed by distillation of the product at 143-151°C and 1mm Hg yielded 12.6g of (CFIC) (white solid, mp: 55.5-56.5°C).

## Claims

1. A method of manufacture of chloroformyloxy-substituted isophthaloyl chloride comprising forming a mixture of hydroxy-substituted isophthalic acid, or salt thereof, catalyst, phosgene and solvent, heating said mixture under autogeneous pressure, treating said mixture to remove said solvent, and distilling said mixture to yield said chloroformyloxy-substituted isophthaloyl chloride.

2. The method of claim 1 wherein said chloroformyloxy-substituted isophthaloyl chloride is selected from 4-chloroformyloxyisophthaloyl chloride, 2-chloroformyloxyisophthaloyl chloride, and 5-chloroformyloxyisophthaloyl chloride.

3. The method of claim 1 or 2 wherein said hydroxy-substituted isophthalic acid is 5-hydroxyisophthalic acid.

4. The method of claims 1, 2 or 3 wherein said catalyst is imidazole.

5. The method of any one of claims 1 to 4 wherein said solvent is chlorinated benzene.

6. The method of claim 5 wherein said chloroformyloxy-substituted isophthaloyl chloride is 5-chloroformyloxyisophthaloyl chloride.
